(19) **Europäisches Patentamt / European Patent Office / Office européen des brevets**

(11) **EP 4 534 644 A1**

(12) **EUROPEAN PATENT APPLICATION**
published in accordance with Art. 153(4) EPC

(43) Date of publication:
09.04.2025 Bulletin 2025/15

(21) Application number: 23880005.6

(22) Date of filing: 01.09.2023

(51) International Patent Classification (IPC):
*C12N 1/12* (2006.01)   *C12N 1/00* (2006.01)

(52) Cooperative Patent Classification (CPC):
C12N 1/00; C12N 1/12

(86) International application number:
PCT/KR2023/013055

(87) International publication number:
WO 2024/085425 (25.04.2024 Gazette 2024/17)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 18.10.2022 KR 20220134302

(71) Applicant: CJ CheilJedang Corporation
Seoul 04560 (KR)

(72) Inventors:
• JEONG, A Young
  Seoul 04560 (KR)
• AN, Jungap
  Seoul 04560 (KR)
• LEE, In
  Seoul 04560 (KR)

(74) Representative: Meissner Bolte Partnerschaft
mbB
Patentanwälte Rechtsanwälte
Postfach 86 06 24
81633 München (DE)

(54) **METHOD FOR PRODUCING MICROALGAE CONCENTRATES WITH LOW VISCOSITY**

(57) The present invention relates to a method for producing a microalgae concentrate with low viscosity, the method comprising a step of adjusting the pH of a microalgae fermentation broth and a heat treatment step, in which utility costs and investment costs can be reduced in a dehydration and drying step to secure price competitiveness for production and commercialization of dried microalgae products.

【FIG. 1】

EP 4 534 644 A1

**Description**

[TECHNICAL FIELD]

Cross-reference to related application(s)

The present application claims the benefit of the priority based on Korean Patent Application No. 10-2022-0134302 filed on October 18, 2022, and the entire contents of the document of the corresponding Korean patent application are incorporated as a part of the present description.

The present invention relates to a method for producing a microalgal concentrate with low viscosity comprising adjusting pH of a microalgal fermented solution and heat-treating.

[BACKGROUND ART]

United Nations Food and Agriculture Organization (FAO) forecasts an increase in meat consumption due to an increase in the world population, and the alternative protein food market is rapidly growing, according to an increase in international interests in environmental issues such as global warming and the like, and shortage in meat supply and price jump due to recent COVID-19. Accordingly, in the middle of conversion from the era of 'cultivation·breeding' to the ear of 'extracting·fermenting·culturing', new business opportunities are also on the rise. In particular, as interests in sustainability in the social and environmental sectors, such as health, environment, animal welfare and the like around the world, a plant-oriented alternative protein market is expanding among various alternative proteins.

As a raw material of vegetable protein, microalgae are emerging. Microalgae are a resource rich in carbohydrates and lipids, and are used as food due to their high content of vitamins and essential fatty acids. They have been used as various animal feed raw materials from aquaculture to livestock farms as they are rich in protein, and about 30% of biomass produced worldwide is solid as animal feed. Microalgae protein is a promising business field with an average annual growth rate of 5.8% expected to increase from 525.40 million dollars in 2018 to about 748.20 million dollars in 2023.

Microalgae have high productivity per unit area, and use of non-cultivated land and unused space is possible, and by use of conventional infrastructure, reduction of social investment costs is possible. **In** addition, it has many advantages in that it is possible to secure high value-added raw materials such as pigments as well as protein sources. However, they have been researched from various researchers and companies for decades, but development of economical systems for industrialization has not been commercialized except for a few examples. Despite the high potential of microalgae, due to limitations of low yield and high production cost, many element technologies for commercialization are only at the research and development stage or empirical research. In order to derive productivity on a commercial scale for production of microalgae-based products, securing competitiveness in the unit cost of production is the most important factor.

In order to economically produce products comprising microalgae, a technology of effectively drying harvested microalgae is required. As particles of microalgae are fine as 3 ~ 30 $\mu$m, drying is not performed well by a general drying method, so a method such as freeze-drying or spray drying or the like with high energy and cost are often used, but in order to produce products to be used in food which are not high value-added products such as medicine, a drying method capable of minimizing use of energy is essential. A natural drying method, hot air drying, a drying method using a drum drier or belt drier, and the like, which are economical drying methods, are used, but even if dried by the above methods, there is a limit to lowering the production cost by using a drying method with steam efficiency which does not exceed 1. Therefore, development of a more efficient low-energy and low-cost drying method is needed.

[DISCLOSURE]

[TECHNICAL PROBLEM]

One embodiment of the present application provides a method for producing a microalgal concentrate with low viscosity, comprising;

    (1) adjusting pH of a microalgal fermented solution; and
    (2) heat-treating the fermented solution.

Another embodiment of the present application provides a method for drying a microalgal fermented solution comprising;

    (1) adjusting pH of a microalgal fermented solution;
    (2) heat-treating the fermented solution;

(3) evaporating and concentrating the heat-treated fermented solution; and
(4) drying the evaporated and concentrated microalgal concentrate.

Other embodiment provides a microalgal concentrate with low viscosity, produced by a method for producing a microalgal concentrate with low viscosity, comprising (1) adjusting pH of a microalgal fermented solution; and (2) heat-treating the fermented solution.

[TECHNICAL SOLUTION]

Each description and embodiment disclosed in the present application can be applied to each of other description and embodiments. In other words, all combinations of various elements disclosed in the present application fall within the scope of the present invention. In addition, it cannot be seen that the scope of the present invention is limited by the detailed description described below. Furthermore, those skilled in the art will recognize or confirm many equivalents to specific aspects of the present invention described in the present application using only a common experiment. In addition, such equivalents are intended to be included in the present invention.

One embodiment of the present application provides a method for producing microalgal concentrate with low viscosity, comprising;

(1) adjusting pH of a microalgal fermented solution; and
(2) heat-treating the fermented solution.

The term used in the present description, "microalgae" means small algae that cannot be seen with naked eyes and can be seen only through a microscope, and various kinds are included in the microalgae, and even strains that cannot photosynthesize and grow only as heterotrophs are included. Such microalgae can fix carbon dioxide and have high protein and lipid contents, so can be used as biomass for food, feed or fuel production. Specifically, the lipid component contained in the microalgae can be used as a raw material for production of biodiesel oil used as liquid fuel.

In the present description, the microalgae may be a *Schizochytrium* sp., *Thraustochytrium* sp., *Aurantiochytrium* sp., *Japonochytrium* sp., *Ulkenia* sp., *Crypthecodinium* sp. or *Haliphthoros* sp. strain, preferably, a *Schizochytrium* sp. or *Thraustochytrium* sp. strain, and most preferably, it may be a *Schizochytrium* sp. strain.

The term used in the present description, "fermented solution" comprises products produced by fermenting the microalgae, and may be interchangeably used with the terms, "fermented stock solution" or "culture solution", and specifically, it may be a fermented solution comprising microalgae or a fermented filtrate in which microalgae are removed from the fermented solution, but not limited thereto.

The microalgal fermented solution may be produced by inoculating the microalgae in a microalgal culture medium according to a culture method known in the art.

The microalgal culture medium may be MJW01 medium, but not limited thereto.

In the present invention, "adjusting pH" of the step (1) may be interchangeably used with the term, "regulating pH", and regulation of pH may be regulating by adding a sulfuric acid solution, but not limited thereto, and any method may be used as long as it is a method capable of regulating pH.

The adjusting pH of the step (1) may be adjusting pH to 4 or less. When the pH of the microalgal fermented solution is adjusted to 5 or more and then it is concentrated, the viscosity of the microalgal concentrate compared to the solid content (%) increases, so the steam efficiency of the drying process after the concentrating process is not good, and therefore, a relatively large steam amount is required.

The heat-treating of the step (2) may be performed at a temperature of 80°C or more, 100°C or more, 120°C or more, 80°C to 150°C, 100°C to 150°C, or 120°C to 150°C.

The heat-treating of the step (2) may be performed for 5 minutes or more, 10 minutes or more, 15 minutes or more, 5 minutes to 60 minutes, 10 minutes to 60 minutes, 5 minutes to 30 minutes, 10 minutes to 30 minutes, 5 minutes to 25 minutes, 10 minutes to 25 minutes, 5 minutes to 20 minutes, or 10 minutes to 20 minutes, but not limited thereto.

The method for producing a microalgal concentrate may further comprise, step (3) concentrating the heat-treated fermented solution after the heat-treating of the step (2).

The concentrating the heat-treated fermented solution may be performed using an evaporation concentrator, but not limited thereto.

Furthermore, the present invention provides a method of drying a microalgal fermented solution comprising;

(1) adjusting pH of a microalgal fermented solution;
(2) heat-treating the fermented solution;
(3) evaporating and concentrating the heat-treated fermented solution; and
(4) drying the evaporated and concentrated microalgal concentrate.

The contents of the steps (1) to (3) are as described above.

The drying the microalgal concentrate of the step (4) may be performed using a drum drier, but not limited thereto, and it may be drying using an appropriate method known in the art.

When the drying the microalgal concentrate of the step (4) is performed using a drum drier, the steam amount required for drying of the process which passes through the steps (1) to (3) of the present application may be 2.3 times or more less than a process which does not pass through the steps (1) to (3).

In addition, the present invention provides a microalgal concentrate with a solid content of the microalgal concentrate of 15 % by weight or more based on the total weight of the microalgal concentrate, and viscosity of 120 cP or less.

The viscosity of the microalgal concentrate may be 120 cP or less, 110 cP or less, or 100cP or less, and the solid content of the microalgal concentrate may be 15 % by weight or more, 17 % by weight or more, 20 % by weight or more, 25 % by weight or more, 30 % by weight or more, 15 to 40 % by weight, 17 to 40 % by weight, 20 to 40 % by weight, 25 to 40 % by weight, 30 to 40 % by weight, 15 to 35 % by weight, 17 to 35 % by weight, 20 to 35 % by weight, 25 to 35 % by weight, or 30 to 35 % by weight, based on the total weight of the microalgal concentrate.

In addition, the present invention provides a microalgal concentrate with a high solid content and low viscosity produced by a method for producing a microalgal concentrate comprising (1) adjusting pH of a microalgal fermented solution; and (2) heat-treating the fermented solution.

The viscosity of the microalgal concentrate produced by the concentration method of the present invention may be 120 cP or less, 110 cP or less, or 100cP or less, and the solid content of the microalgal concentrate may be 15 % by weight or more, 17 % by weight or more, 20 % by weight or more, 25 % by weight or more, 30 % by weight or more, 15 to 40 % by weight, 17 to 40 % by weight, 20 to 40 % by weight, 25 to 40 % by weight, 30 to 40 % by weight, 15 to 35 % by weight, 17 to 35 % by weight, 20 to 35 % by weight, 25 to 35 % by weight, or 30 to 35 % by weight, based on the total weight of the microalgal concentrate.

In the examples of the present invention, it was confirmed that the microalgal concentrate concentrated by passing through a pretreatment process comprising heat-treating, after adjusting the pH of a microalgal fermented solution to 4 or less had characteristics including low viscosity and a high solid content, so the method for producing a microalgal concentrate of the present invention can be usefully used as a pretreatment process of a drying method with low energy and low cost, as it can reduce a steam amount required in the subsequent drying step.

[ADVANTAGEOUS EFFECTS]

The present invention relates to a method for producing a microalgal concentrate with low viscosity comprising adjusting pH of a microalgal fermented solution and heat-treating, and provides a method for reducing utility costs and investment costs in dehydration and drying processes in order to secure price competitiveness for commercialization of production of microalgal drying products. Specifically, in the method for producing a microalgal concentrate of the present invention, the microalgal concentrate which passes through pretreatment processes of pH adjustment and heat-treatment in a range without crushing a microalgal strain has low viscosity, so it has physical properties capable of discharge and transport. As a general microalgal fermented solution has high viscosity, due to the characteristic that the viscosity increases even after a small amount of dehydration process and fine cell size, it essentially requires filtration, a mechanical separator, a dehydration process through evaporation and concentration processes of which process costs are relatively inexpensive. The present invention has confirmed conditions of a pretreatment process capable of producing a microalgal concentrate with the lowest viscosity in a microalgal concentration process, and through this, it improves that use of a conventional multiple condensation tube with high steam efficiency is impossible, and finally, it can reduce utility costs through steam cost reduction and reduce investment costs of a drier in a drying process.

[BRIEF DESCRIPTION OF THE DRAWINGS]

FIG. 1 is a diagram showing the viscosity depending on the solid content (%) of the microalgal concentrate concentrated by adjusting a microalgal fermented solution to a variety of pH (pH= 3, 4, 5, 6) under the same temperature condition.

FIG. 2 is a diagram showing the viscosity depending on the solid content (%) of the microalgal concentrate concentrated by heat-treating a microalgal fermented solution at various temperatures under a pH non-adjusted condition.

FIG. 3 is a diagram showing the viscosity depending on the solid content (%) of the microalgal concentrate concentrated by adjusting the pH of a microalgal fermented solution to 5 and then heat-treating at various temperatures.

FIG. 4 is a diagram showing the viscosity depending on the solid content (%) of the microalgal concentrate concentrated by adjusting the pH of a microalgal fermented solution to 5, 4, 3.5 or 3, and then heat-treating at 120°C.
FIG. 5 is a diagram showing the viscosity depending on the solid content (%) of the microalgal concentrate concentrated by adjusting the pH of a microalgal fermented solution to 4, and then heat-treating at various temperatures.
FIG. 6 is a diagram showing the direct drying process chart and pretreatment drying process chart.

[MODE FOR INVENTION]

Hereinafter, the present invention will be described in more detail by the following examples. However, these examples are intended to illustrate the present invention only, but the scope of the present invention is not limited by these examples.

For product quality, conditions, in which solids after evaporation and concentration are 30% or more, and the viscosity is 100cP or less, which is capable of discharging in an evaporation concentrator, were searched through adjusting pH and heat-treating under a condition in which cells are not crushed.

**Example 1. Preparation of microalgal fermented solution**

In order to secure a microalgal fermented solution for an experiment, a *Schizochytrium* fermented solution was recovered from a fermenter. Specifically, culturing a *Schizochytrium* sp. strain was performed in a 5L fermenter by supplying a glucose carbon source of 25% compared to the total culture solution for 30 hours. On a purpose of seed culture, using a sterilized MJW01 medium, culture was conducted in a 500mL flask under conditions of 30°C, 150 rpm for about 20 hours. The seed cultured flask was aliquoted and inoculated in a 5L fermenter, and culturing was performed under conditions of the sterilized MJW01 medium and culture environment 30°C, 500 rpm, 1.5 vvm, pH 5-8.

**Experimental example 1. Confirmation of viscosity of microalgal concentrate in which microalgal fermented solution is adjusted to various pHs under the same temperature condition**

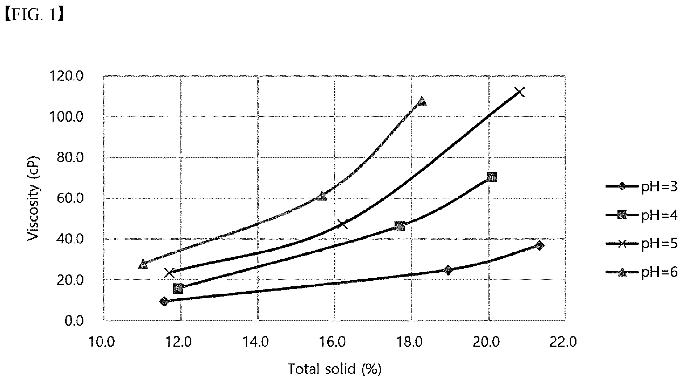

pH was adjusted to 3, 4 or 5 by adding 98% sulfuric acid solution to a microalgal fermented solution with a solid content of 8% after completing culturing under conditions of pH=6, 30°C. The fermented solution in which the pH was adjusted was added to an evaporation concentrator (Eyela company( 社) N-1210B) by 1L each, and moisture was evaporated under a condition of an internal temperature of about 70°C to evaporate and concentrate. After that, the viscosity by solid content (%) of each microalgal concentrate was measured, and the result was shown in Table 1 and FIG. 1 below.

The solid content (%) was measured by the following method. With a principle of measuring a ratio of solids remained after heating a sample to be measured and drying moisture, a sample bottle was dried at 105°C for about 1 hour, and then cooled in a desiccator, and then the weight was measured immediately before use. Then, after measuring the weight of the sample bottle and sample accurately after taking an appropriate amount of the sample, they were completely dried in a 105°C drier for 3 hours, and then cooled by placing in a desiccator containing silica gel, and then the weight was measured. The measured value was substituted in the following equation to calculate the solid content (Total solid %).

[Equation]

$$\text{Total solid } \% = \frac{W_3 - W_1}{W_2 - W_1} \times 100$$

* $W_1$ = Weight of sample bottle
* $W_2$ = Weight of sample bottle and sample before drying
* $W_3$ = Weight of sample bottle and sample after drying

For viscosity (cP), the viscosity of the microalgal fermented solution and concentrate was measured under conditions of 70°C, 1000 shear rate using a viscosity measurement equipment (RheolabQC, Anton paar company ( 社)).

**[Table 1]**

| Adjusted pH | pH=3 | | pH=4 | | pH=5 | | pH=6 | | pH 7 | |
|---|---|---|---|---|---|---|---|---|---|---|
| Analysis item | Solid | Viscosity | Solid | Viscosity | Solid | Viscosity | Solid | Viscosity | Solid | Viscosity |
| Unit | % | cP | % | cP | % | cP | % | cP | % | cP |
| Value | 11.6 | 9.3 | 11.9 | 15.7 | 11.7 | 23.5 | 11.0 | 27.8 | 11.2 | 27.9 |
| | 19.0 | 24.8 | 17.7 | 46.2 | 16.2 | 47.3 | 15.7 | 61.4 | 16.0 | 60.6 |
| | 21.3 | 36.8 | 20.1 | 70.3 | 20.8 | 112.1 | 18.3 | 107.7 | 18.6 | 108.7 |

As a result, as shown in Table 1 and FIG. 1, it was confirmed that the viscosity compared to the solid content (%) of the same microalgal concentrate was lower, as the pH was lower, and it was confirmed that the solid content (%) of the microalgal concentrate with viscosity at a degree capable of discharging in a concentrator (about 100cP) increased.

**Experimental example 2. Confirmation of viscosity of microalgal concentrate in which microalgal fermented solution is heat-treated at various temperatures under pH non-adjusted condition**

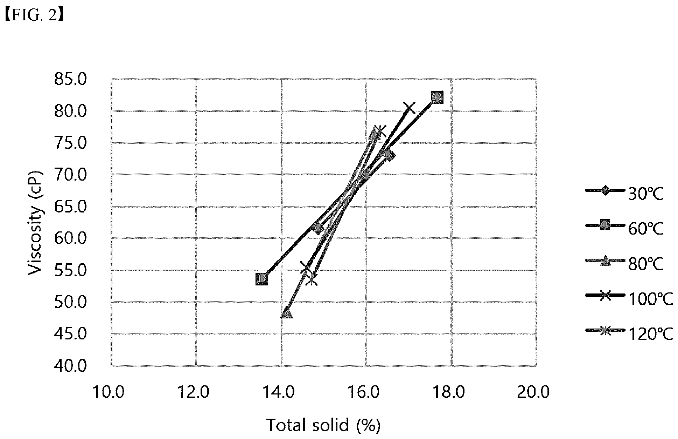

The microalgal fermented solution with a solid content of 8% in which culturing was completed under conditions of pH=6, 30°C was heat-treated at various temperatures (30°C, 60°C, 80°C, 100°C and 120°C) for about 15 minutes. The fermented solution heat-treated at temperatures different from each other was put into an evaporation concentrator (Eyela company (社) N-1210B) by 1L each to evaporate moisture, and the viscosity by solid content (%) of the microalgal concentrate was measured, and the result was shown in Table 2 and FIG. 2 below.

**[Table 2]**

| pH | 6 | | 6 | | 6 | | 6 | | 6 | |
|---|---|---|---|---|---|---|---|---|---|---|
| Temperature | 30°C | | 60°C | | 80°C | | 100°C | | 120°C | |
| Analysis item | Solid | Viscosity | Solid | Viscosity | Solid | Viscosity | Solid | Viscosity | Solid | Viscosity |
| Unit | % | cP | % | cP | % | cP | % | cP | % | cP |
| Value | 14.9 | 61.5 | 13.5 | 53.6 | 14.1 | 48.4 | 14.6 | 55.4 | 14.7 | 53.5 |
| | 16.6 | 73.0 | 17.7 | 82.2 | 16.2 | 76.4 | 17.0 | 80.5 | 16.3 | 76.8 |

As a result, as shown in Table 2 and FIG. 2, it was confirmed that there was no tendency for viscosity change by solid content (%) depending on heat-treatment temperature, when the microalgal fermented solution in which culturing was completed was heat-treated without passing through adjusting pH.

**Experimental example 3. Confirmation of viscosity of microalgal concentrate in which microalgal fermented solution is heat-treated at various temperatures under condition in which pH is adjusted to 5**

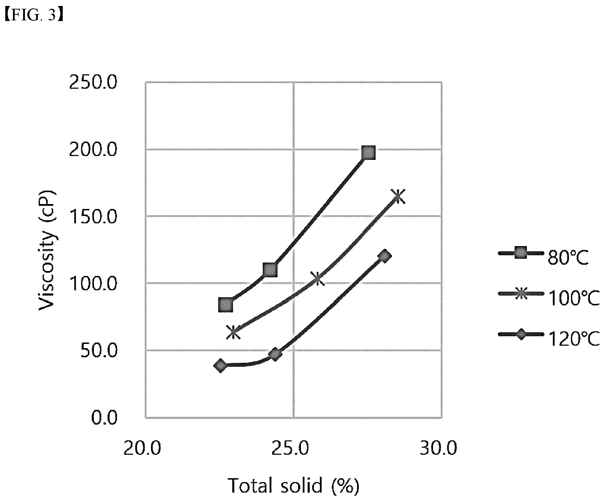

pH was adjusted to 5, by adding 98% sulfuric acid to the microalgal fermented solution with a solid content of 8% in which culturing was completed under conditions of pH=6, 30°C. After that, each 1L of the microalgal fermented solution was heat-treated at various temperatures (80°C, 100°C and 120°C) for about 15 minutes. The microalgal fermented solution heat-treated at temperatures different from each other was put into an evaporation concentrator (Eyela company (社) N-1210B) by 800g each to evaporate moisture, and the viscosity by solid content (%) was measured and shown in Table 3 and FIG. 3 below.

**[Table 3]**

| Adjusted pH | pH=5 | | pH=5 | | pH=5 | |
|---|---|---|---|---|---|---|
| Temperature | 80°C | | 100°C | | 120°C | |
| Time | 15min | | 15min | | 15min | |
| Analysis item | Solid | Viscosity | Solid | Viscosity | Solid | Viscosity |
| Unit | % | cP | % | cP | % | cP |
| Value | 22.7 | 84.3 | 23.0 | 63.6 | 22.5 | 38.5 |
| | 24.2 | 110.4 | 25.8 | 103.5 | 24.4 | 47.2 |
| | 27.5 | 197.4 | 28.5 | 165.2 | 28.1 | 120.6 |

As a result, as shown in Table 3 and FIG. 3, it was confirmed that the higher the heat-treatment temperature, the lower the viscosity based on the solid content (%), when adjusting pH to 5 was passed through before heat-treatment. However, there was a limit that the solid content of microalgal concentrates that passed through pretreatment of adjusting pH to 5 did not exceed 30%.

**Experimental example 4. Confirmation of viscosity of microalgal concentrate in which microalgal fermented solution is heat-treated at the same temperature under various pH conditions**

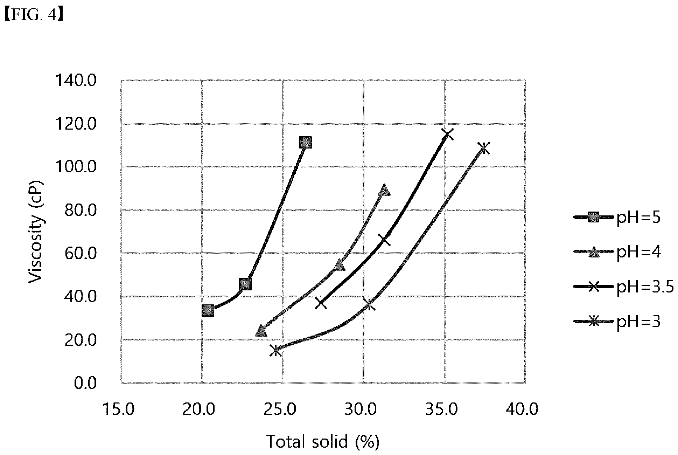

pH was adjusted to 5, 4, 3.5 or 3, by adding 98% sulfuric acid to the microalgal fermented solution with a solid content of 8% in which culturing was completed under conditions of pH=6, 30°C. After that, each 1L of the microalgal fermented solution was heat-treated at the temperature of 120°C for about 15 minutes. The microalgal fermented solution heat-treated was put into an evaporation concentrator (Eyela company ( 社) N-1210B) by 800g each to evaporate moisture, and the viscosity by solid content (%) was measured and shown in Table 4 and FIG. 4 below. In addition, in order to confirm the viscosity of the microalgal concentrate heat-treated at the same temperature under a relatively high pH condition, the result of adjusting the pH of the fermented solution to 6 or 7, and treating under the same condition was shown in Table 5 below.

**[Table 4]**

| Adjusted pH | pH=5 | | pH=4 | | pH=3.5 | | pH=3 | |
|---|---|---|---|---|---|---|---|---|
| Temperatur e | 120°C | | 120°C | | 120°C | | 120°C | |
| Time | 15min | | 15min | | 15min | | 15min | |
| Analysis item | Solid | Viscosity | Solid | Viscosity | Solid | Viscosity | Solid | Viscosit y |
| Unit | % | cP | % | cP | % | cP | % | cP |
| Value | 20.4 | 33.2 | 23.7 | 24.4 | 27.4 | 36.9 | 24.6 | 15.1 |
| | 22.7 | 45.5 | 28.5 | 54.7 | 31.3 | 66.2 | 30.4 | 36.3 |
| | 26.5 | 111 | 31.3 | 89.4 | 35.2 | 115.2 | 37.4 | 108.6 |

**[Table 5]**

| Adjusted pH | pH=6 | | pH=7 | |
|---|---|---|---|---|
| Temperature | 120°C | | 120°C | |
| Time | 15min | | 15min | |
| Analysis item | Solid | Viscosity | Solid | Viscosity |
| Unit | % | cP | % | cP |
| Value | 19.8 | 113.4 | 20.3 | 110.5 |
| | 24.1 | 222.7 | 24.7 | 246.2 |

As a result, as shown in Table 4 and FIG. 4, it was confirmed that as the pH was lowered before the heat-treating process of the microalgal fermented solution, there was an effect that the fermented solution was concentrated and the viscosity was lowered. On the other hand, as shown in Table 5, as the viscosity was shown higher when the pH before the heat-treating process of the microalgal fermented solution was adjusted to 6 or 7, compared to that when it was adjusted to low pH, it was confirmed that the fermented solution was not sufficiently concentrated.

In order to achieve economic feasibility in the microalgal concentration process, it was confirmed that the solid content (%) of the microalgal concentrate was shown as a value of 30% or more in an experimental group in which the solid content (%) of a target concentrate was 30% or more, and the pH before heat-treatment of the microalgal fermented solution was adjusted to 4 or less, so the condition in which the pH before heat-treatment was adjusted to 4 or less was established as a pretreatment condition producing a microalgal concentrate with a high solid content and low viscosity.

**Experimental example 5. Confirmation of viscosity of microalgal concentrate in which microalgal fermented solution is heat-treated at various temperatures under condition in which pH is adjusted to 4**

As the condition in which the pH was adjusted to 4 or less was derived as a pretreatment condition to produce a microalgal concentrate with a high solid content and low viscosity in the Experimental example 4, in order to derive an optimal heat-treatment temperature for producing a microalgal concentrate with viscosity lower than the solid content (%) under a condition in which the pH of the microalgal fermented solution was adjusted to 4, the following experiment was performed.

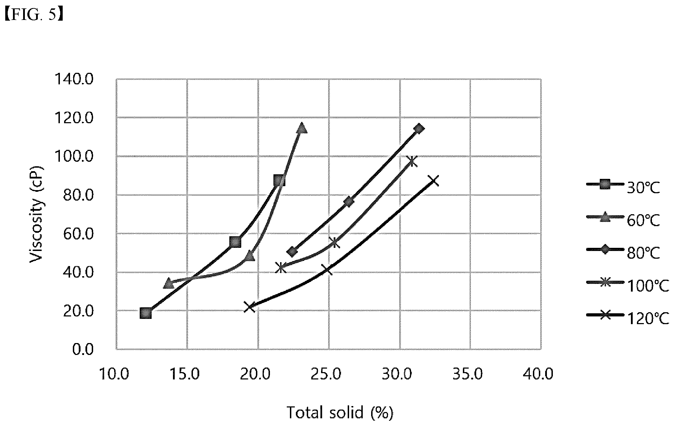

Specifically, the pH of the fermented solution was adjusted to 4 by adding 98% sulfuric acid to the microalgal fermented solution in which the solid content was 8% after culturing was completed under conditions of pH=6, 30°C. After that, each 1L of the microalgal fermented solution was heat-treated at a temperature of 30°C, 60°C, 80°C, 100°C or 120°C for about 15 minutes. The heat-treated fermented solution was put into an evaporation concentrator (Eyela company (社) N-1210B) by 800g each to evaporate moisture, and the viscosity by solid content (%) was measured and shown in Table 6 and FIG. 5 below.

[Table 6]

| pH | 4 | | 4 | | 4 | | 4 | | 4 | |
|---|---|---|---|---|---|---|---|---|---|---|
| Temperatu re | 30°C | | 60°C | | 80°C | | 100°C | | 120°C | |
| Time | 15min | | 15min | | 15min | | 15min | | 15min | |
| Analysis item | Soli d | Viscosi ty | Soli d | Viscosi ty | Soli d | Viscosi ty | Soli d | Viscosi ty | Soli d | Viscosi ty |
| Unit | % | cP | % | cP | % | cP | % | cP | % | cP |
| Value | 12.1 | 18.6 | 13.7 | 34.3 | 22.4 | 50.4 | 21.6 | 42.3 | 19.4 | 21.9 |
| | 18.4 | 55.4 | 19.4 | 48.7 | 26.4 | 76.4 | 25.4 | 55.3 | 24.9 | 41.3 |
| | 21.6 | 87.4 | 22.4 | 98.9 | 29.5 | 99.1 | 30.9 | 97.4 | 32.4 | 87.2 |

As a result, as shown in Table 6 and FIG. 5, it was confirmed that the higher the heat-treatment temperature, the lower the viscosity based on the same solid content (%) under a condition in which the pH of the microalgal fermented solution was adjusted to 4. Specifically, the viscosity data did not increase linearly with the change in solid content, but increased rapidly when it became a certain concentration or more quadratically, and it was confirmed that the solid content could be further concentrated by 30% or more when heat-treated at a temperature of 80°C or more, as the solid content was 23% at 60°C and 31% at 80°C based on the same 114 cp. Therefore, it is determined that performing heat-treatment at a temperature of 80°C or more has a better concentration effect.

**Experimental example 6. Confirmation of steam efficiency depending on pH adjusting and heat-treating pretreatment process before drying**

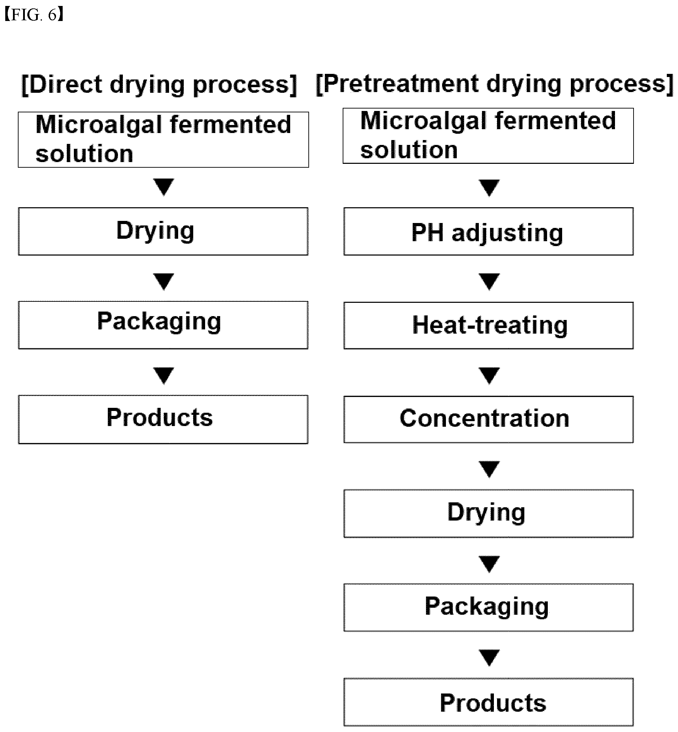

The total amount of steam used was calculated through material balance writing based on the same fermentation index and produced amount for the microalgal products dried after passing through a pretreatment process of pH adjusting and heat-treating, and microalgal products directly dried without the pretreatment process, and shown in Table 7 below. The direct drying process chart and the process chart of drying after pretreatment were shown in FIG. 6.

**[Table 7]**

| Process condition Unit Process | Direct drying Used amount (Ton/day) | Pretreatment drying (pH adjusting and heat-treating) Used amount (Ton/day) |
|---|---|---|
| Heat-treatment steam | - | 64.50 |
| Concentration tube steam | - | 118.20 |
| Drier steam | 1,374.56 | 421.30 |
| Total | **1,374.56** | **604.00** |

As a result, as shown in Table 7, it was confirmed that the used amount of steam was reduced by about 2.3 times when drying using a drum drier after evaporation and concentration by passing through pH adjusting and heat-treating, compared to drying (direct drying) using a drum drier without a pretreatment process for the microalgal fermented solution. As a result of comparing the used amount of drier steam, the amount of steam required when the pretreatment process was passed through was 421.3 ton/day, and the amount of steam required when the microalgal fermented solution was put into a drier without the pretreatment process was shown as 1,374.6 ton/day. In other words, since the amount of steam required for the pretreatment process drying was about 3 times less than that of the direct drying, the number of driers required can be reduced by about 3 times.

**Experimental example 7. Confirmation of number of cells of microalgal fermented solution and concentrate passing through pretreatment process**

In order to confirm whether strain characteristics were maintained even after pretreatment, the number of the microalgal cells of the microalgal fermentation stock solution and the microalgal process solution (concentrate) passing through the pretreatment process (pH adjusting and heat-treating) was measured using Anvajo company(社), fluidlab R-300 equipment, and shown in Table 8.

**[Table 8]**

| Process solution | Fermentation stock solution | Pretreatment process solution |
|---|---|---|
| BT1 | 2.46E+08 | 2.48E+08 |
| BT2 | 3.59E+08 | 3.35E+08 |
| BT3 | 2.19E+08 | 2.22E+08 |

As a result, as shown in Table 8, it was confirmed that the change in the number of cells between the microalgal fermentation stock solution and the process solution (concentrate) which passed through the pretreatment process was not large, so it was confirmed that microalgal strain characteristics were maintained even through the pretreatment process.

**Claims**

1.    A method for producing a microalgal concentrate with low viscosity, comprising;

　　　(1) adjusting pH of a microalgal fermented solution; and
　　　(2) heat-treating the fermented solution.

2.    The method for producing a microalgal concentrate according to claim 1, wherein the microalgae is a *Schizochytrium* sp. strain.

3.    The method for producing a microalgal concentrate according to claim 1, wherein the (1) adjusting pH is adjusting pH to 4 or less.

4.    The method for producing a microalgal concentrate according to claim 1, wherein the (2) heat-treating is performed at a temperature of 80°C or more.

5. The method for producing a microalgal concentrate according to claim 1, wherein the (2) heat-treating is performed at a temperature of 100°C or more.

6. The method for producing a microalgal concentrate according to claim 1, wherein the (2) heat-treating is performed for 5 minutes to 25 minutes.

7. The method for producing a microalgal concentrate according to claim 1, wherein the solid content of the microalgal concentrate is 15 % by weight based on the total weight of the microalgal concentrate.

8. The method for producing a microalgal concentrate according to claim 1, wherein the viscosity of the microalgal concentrate is 120 cP or less.

9. A method for drying a microalgal fermented solution comprising;

    (1) adjusting pH of a microalgal fermented solution;
    (2) heat-treating the fermented solution;
    (3) evaporating and concentrating the heat-treated fermented solution; and
    (4) drying the evaporated and concentrated microalgal concentrate.

10. A microalgal concentrate, in which the solid content of the microalgal concentrate is 15 % by weight based on the total weight of the microalgal concentrate, and the viscosity is 120 cP or less.

11. The microalgal concentrate according to claim 10, wherein the microalgal concentrate is produced by the method of claim 1.

【FIG. 1】

【FIG. 2】

【FIG. 3】

【FIG. 4】

【FIG. 5】

【FIG. 6】

**[Direct drying process]**   **[Pretreatment drying process]**

| Microalgal fermented solution |
| :---: |

▼

| Drying |
| :---: |

▼

| Packaging |
| :---: |

▼

| Products |
| :---: |

| Microalgal fermented solution |
| :---: |

▼

| PH adjusting |
| :---: |

▼

| Heat-treating |
| :---: |

▼

| Concentration |
| :---: |

▼

| Drying |
| :---: |

▼

| Packaging |
| :---: |

▼

| Products |
| :---: |

## INTERNATIONAL SEARCH REPORT

| | International application No. |
|---|---|
| | **PCT/KR2023/013055** |

| A. | CLASSIFICATION OF SUBJECT MATTER |
|---|---|

**C12N 1/12**(2006.01)i; **C12N 1/00**(2006.01)i

According to International Patent Classification (IPC) or to both national classification and IPC

| B. | FIELDS SEARCHED |
|---|---|

Minimum documentation searched (classification system followed by classification symbols)

C12N 1/12(2006.01); C11B 1/00(2006.01); C11B 1/02(2006.01); C12N 1/14(2006.01); C12P 7/64(2006.01); C12P 7/6434(2022.01); C12R 1/89(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 미세조류(microalgae), 스키조키트리움(Schizochytrium), 발효(fermentation), pH, 열(heat), 증발농축(concentration by evaporation), 점도(viscosity)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 104371926 A (CHINA PETROLEUM & CHEMICAL CORP. et al.) 25 February 2015 (2015-02-25) See paragraphs [0019], [0021], [0024], [0027], [0082] and [0083]. | 1-8,10,11 |
| Y | | 9 |
| Y | JP 2019-017328 A (BIOX CHEM INDUSTRIES CO., LTD. et al.) 07 February 2019 (2019-02-07) See paragraph [0046]. | 9 |
| A | WO 2017-203959 A1 (KAO CORPORATION) 30 November 2017 (2017-11-30) See entire document. | 1-11 |
| A | CN 103173273 A (ENN RESEARCH & DEVELOPMENT CO., LTD.) 26 June 2013 (2013-06-26) See entire document. | 1-11 |
| A | CN 115058462 A (NOVOSANA (TAICANG) CO., LTD.) 16 September 2022 (2022-09-16) See entire document. | 1-11 |

| ☐ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **30 November 2023** | **30 November 2023** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

# EP 4 534 644 A1

**INTERNATIONAL SEARCH REPORT**
**Information on patent family members**

| International application No. |
| --- |
| **PCT/KR2023/013055** |

| Patent document cited in search report | | | Publication date (day/month/year) | Patent family member(s) | | | Publication date (day/month/year) |
| --- | --- | --- | --- | --- | --- | --- | --- |
| CN | 104371926 | A | 25 February 2015 | None | | | |
| JP | 2019-017328 | A | 07 February 2019 | JP | 6647593 | B2 | 14 February 2020 |
| WO | 2017-203959 | A1 | 30 November 2017 | AU | 2017-270156 | A1 | 30 November 2017 |
| | | | | JP | 2017-209101 | A | 30 November 2017 |
| | | | | JP | 6985792 | B2 | 22 December 2021 |
| | | | | US | 2019-0292512 | A1 | 26 September 2019 |
| CN | 103173273 | A | 26 June 2013 | CN | 103173273 | B | 25 November 2015 |
| CN | 115058462 | A | 16 September 2022 | None | | | |

Form PCT/ISA/210 (patent family annex) (July 2022)

**REFERENCES CITED IN THE DESCRIPTION**

**Patent documents cited in the description**

- KR 1020220134302